(19) Europäisches Patentamt
European Patent Office
Office européen des brevets

(11) Numéro de publication: **0 157 684**
**A1**

(12) **DEMANDE DE BREVET EUROPEEN**

(21) Numéro de dépôt: **85400499.1**

(22) Date de dépôt: **14.03.85**

(51) Int. Cl.⁴: **C 07 C 77/02**, C 07 C 76/04, C 10 L 1/22

(30) Priorité: **20.03.84 FR 8404423**

(43) Date de publication de la demande: **09.10.85 Bulletin 85/41**

(84) Etats contractants désignés: **BE DE GB NL**

(71) Demandeur: **INSTITUT FRANCAIS DU PETROLE, 4, Avenue de Bois-Préau, F-92502 Rueil-Malmaison (FR)**

(72) Inventeur: **Born, Maurice, 72, rue du Vieux Pont, F-92000 Nanterre (FR)**
Inventeur: **Guibet, Jean-Claude, 5 bis, rue Quinault, F-78100 Saint Germain En Laye (FR)**
Inventeur: **Jersale, Marie-Françoise, 2, rue de la Ronce, F-92410 Ville D'Avray (FR)**
Inventeur: **Lallement, Jacques, 84, 86, Boulevard Félix Faure, F-93300 Aubervilliers (FR)**

(54) **Composés oraniques à fonction nitrate utilisables comme additifs pour carburants diesel.**

(57) On décrit des composés organiques à fonction nitrate utilisables notamment comme additifs pour améliorer l'indice de cétane des carburants Diesel.

Les composés considérés répondent à la formule générale:

$$R-(O-CH_2-CHX)_n-NO_2$$

dans laquelle R représente un radical alkyle, linéaire ou ramifié, de 6 à 20 atomes de carbone, ou un radical aryle substitué, X est un atome d'hydrogène ou un groupe méthyle et n peut prendre une valeur de 1 jusqu'à environ 15.

Ces composés, ajoutés aux carburants pour moteurs Diesel, par exemple à une concentration de 0,01 à 3% en poids, en améliorent l'indice de cétane et permettent le maintien de la propreté des systèmes d'alimentation.

EP 0 157 684 A1

L'invention concerne des composés organiques à fonction nitrate, utilisables principalement comme additifs pour améliorer l'indice de cétane des carburants pour moteurs Diesel et leur aptitude à maintenir propres les systèmes d'injection de ces moteurs.

Le développement rapide d'unités de conversion des fractions lourdes (craquage catalytique, visco-réduction, cokage) fournit des gaz et des essences facilement valorisables, mais également des coupes moyennes (Light Cycle Oil ou L.C.O.) qui ne peuvent, dans un proche avenir, trouver d'autres débouchés que celui consistant à les incorporer dans le pool gazole. On utilise d'ailleurs déjà actuellement en Europe et en particulier en France des gazoles renfermant des proportions variables, mais notables, (5 à 30 %) de L.C.O.

Ces produits se caractérisent surtout par un faible indice de cétane (en général d'environ 40 à 48), une teneur relativement élevée en aromatiques et une tendance à la formation de dépôts à température élevée.

Sur les véhicules à moteur Diesel et plus particulièrement sur ceux qui sont équipés de moteurs à injection indirecte (avec préchambre), les gazoles renfermant des effluents de craquage entraînent une forte émission de bruit, surtout lors des phases de démarrage et de mise en action. Ces inconvénients sont liés directement à l'indice de cétane faible que présentent ces produits.

D'autre part, ces gazoles forment des dépôts sur les aiguilles d'injecteur de moteurs à préchambre, et dans les orifices d'injecteur des moteurs à injection directe. Il n'est plus possible dans ces conditions de maîtriser le débit de carburant injecté en fonction du temps, et l'on constate :

- une grande difficulté, voire une impossibilité, à obtenir un compromis rendement-pollution satisfaisant ;
- un fonctionnement erratique au ralenti et à faible charge avec émission de produits partiellement imbrûlés (fumées bleues, aldéhydes, oxyde de carbone, etc).

Ces types d'inconvénients concernent non seulement les gazoles de craquage, mais aussi ceux obtenus par distillation directe de pétroles bruts à caractère aromatique (Nigéria par exemple).

Pour améliorer l'indice de cétane et le comportement des gazoles, il a été proposé dans l'art antérieur d'utiliser des additifs nitrates d'alkyle, du type nitrate d'isoamyle, d'hexyle ou d'isooctyle, à une dose moyenne de l'ordre de 2000 ppm. Certains produits de cette famille sont commercialiés par la Société Ethyl sous le nom de DII (Diesel Ignition Improvers).

On a maintenant découvert de nouveaux composés à fonction nitrate agissant comme additifs d'amélioration de l'indice de cétane des carburants Diesel et qui présentent en outre des propriétés détergentes vis-à-vis des dispositifs d'injection des moteurs Diesel. Ils permettent en outre une réduction importante des fumées noires, lors des phases de fonctionnement du moteur à pleine puissance.

D'une manière générale, les composés de l'invention peuvent être définis comme répondant à la formule générale.

$$R \left( O-CH_2-CHX \right)_n O-NO_2 \quad (I)$$

dans laquelle R représente un radical alkyle, linéaire ou ramifié, renfermant par exemple de 6 à 20 atomes de carbone, ou un radical aryle substitué par une ou plusieurs (par exemple 2) chaînes alkyles, renfermant par exemple de 4 à 18 atomes de carbone ; X est un atome d'hydrogène ou un groupe méthyle ; et la valeur de n peut aller par exemple de 1 à environ 15, de préférence de 2 à 10.

Les composés de l'invention consistent donc en des nitrates de monoalcools et en des nitrates de mono-et/ou di-alkylphénols polyéthoxylés ou polypropoxylés. Leur masse molaire peut être par exemple d'environ 200 à 1200 et de préférence de 200 à 650.

Les composés de l'invention peuvent contenir uniquement des motifs éthoxylés ou uniquement des motifs propoxylés. On envisage aussi le cas des

composés contenant à la fois des motifs éthoxylés et propoxylés, répartis au hasard ou séquencés.

Parmi les composés selon l'invention, on considère d'une part ceux qui répondent à la formule générale (I) dans laquelle le radical R est le radical alkyle d'un monoalcool aliphatique saturé R-OH, qui peut être d'origine naturelle ou synthétique. On peut citer par exemple le n-hexanol, le n-octanol-1, l'octanol-2, l'éthyl-2 hexanol-1 (provenant du butyraldéhyde), les alcools caprylique, caprique, undécylique, laurique, tridécylique, myristique, pentadécylique, cétylique, heptadécylique, stéarylique, nonadécylique, eicosylique. On peut, bien entendu, utiliser des mélanges de deux ou plusieurs de ces alcools, tels que les octanols et nonanols provenant de la synthèse oxo, les alcools dérivés du procédé ALFOL, par exemple les ALFOLS 6-8, 610, 810, 1012, 1014, 1214, 1216P, 1218, 1412H, 1618 et 1620, commercialisés par la Société CONDEA. On peut encore citer les alcools qui proviennent de la réduction de coupes d'acides gras ayant un nombre d'atomes de carbone compris dans l'intervalle approprié.

Parmi les composés selon l'invention, on considère d'autre part ceux dont le radical R provient de monophénols substitués, tels que par exemple le butylphénol, l'hexylphénol, l'octylphénol, le nonylphénol, le dodécylphénol, l'hexadécylphénol ou l'octadécylphénol, dans lesquels le substituant alkyle est en général en para.

Les composés de l'invention peuvent être préparés selon différentes méthodes de nitration au moyen d'acide nitrique et de composés hydroxylés appropriés, en particulier par des méthodes mettant en oeuvre des agents deshydratants tels que par exemple l'acide sulfurique ou l'anhydride acétique, à des températures comprises entre - 10°C et + 30°C. Les nitrates organiques ainsi obtenus présentent un excès d'acidité, qui peut être facilement neutralisé par exemple au moyen d'une base minérale (soude, potasse ou ammoniaque par exemple). Le rendement de la nitration peut atteindre par exemple 85 %.

Les composés de l'invention sont utilisables comme additifs améliorant l'indice de cétane des carburants pour moteurs Diesel. Dans cette application, ils peuvent être ajoutés aux carburants en des proportions qui peuvent aller de 0,01 à 3 % en poids.

Les carburants Diesel peuvent consister en des coupes de distillation directe de pétroles bruts (notamment à caractère aromatique) ou en des coupes moyennes de conversion de fractions lourdes (L.C.O.), ou encore en des mélanges de coupes de ces divers types. Ils peuvent contenir en outre des proportions mineures (par exemple jusqu'à 25 % en poids) de constituants oxygénés, tels que, par exemple des alcools (notamment de l'éthanol), des cétones ou encore des éthers.

L'addition des composés de l'invention dans les carburants Diesel, par exemple dans les proportions indiquées plus haut, permet d'en élever l'indice de cétane d'une manière significative (par exemple de 2 à 15 points), ainsi que de leur conférer une activité détergente notable, se traduisant dans la pratique par un meilleur maintien de l'état de propreté des circuits d'alimentation et des injecteurs de moteurs Diesel.

Cette action bénéfique se manifeste notamment dans les moteurs Diesel d'automobiles à injection indirecte (préchambre) dans lesquels les aiguilles d'injecteurs sont particulièrement sensibles au phénomène d'encrassement.

Les exemples suivants illustrent l'invention sans la limiter. Dans chacun des exemples 1 à 9, le mélange nitrant utilisé est constitué de 2,25 moles d'acide nitrique et de 4,5 moles d'anhydride acétique par mole de composé hydroxylé.

**EXEMPLE 1**

On utilise comme composé hydroxylé le produit d'addition d'une coupe d'alcools en $C_6$-$C_8$ sur 2 moles d'oxyde d'éthylène (ALFOL 68-2 EO vendu par la Société CONDEA) répondant à la formule

$$C_6H_{13}/C_8H_{17} \leftarrow (OCH_2CH_2)_2 OH.$$

On introduit goutte à goutte en une heure 20,3 g de ce composé (0,1 mole) dans un mélange nitrant agité, constitué de 14,92 g d'acide nitrique fumant à 95 % et de 45,9 g d'anhydride acétique, la température étant maintenue entre - 4° C et 0° C au moyen d'un bain réfrigérant.

On ajoute ensuite très rapidement dans le milieu réactionnel 200 $cm^3$ d'eau glacée. La phase organique qui se sépare est lavée avec 200 $cm^3$ d'eau ammoniacale, puis à l'eau. Après décantation, la phase organique récupérée est séchée sur $Na_2SO_4$ anhydre puis filtrée.

On récupère finalement 20,1 g de produit, soit un rendement réactionnel de 81 % environ.

Le spectre infra-rouge et la teneur en azote du nitrate obtenu (N = 6 %, théorie = 5,65 %) confirment la nature du produit attendu.

$$C_6H_{13}/C_8H_{17}\text{-}(O\text{-}CH_2\text{-}CH_2)_2\text{-}O\text{-}NO_2$$

EXEMPLE 2

On utilise comme composé hydroxylé le produit d'addition de l'alcool laurique sur 4 moles d'oxyde d'éthylène (CEMULSOL LA 40 vendu par la Société SFOS) répondant à la formule $C_{12}H_{25}\text{-}(OCH_2CH_2)_4\text{-}OH$.

Dans des conditions opératoires identiques à celles de l'exemple 1, on effectue la nitration de 31,2 g de CEMULSOL LA 40 (0,1 mole) ; On récupère finalement 34,6 g de produit, soit un rendement réactionnel de 85 % environ.

Le spectre infra-rouge et la teneur en azote du nitrate obtenu (N = 3,7 %, théorie = 3,44 %) confirme la nature du produit attendu.

$$C_{12}H_{25}\text{-}(O\text{-}CH_2\text{-}CH_2)_4\text{-}O\text{-}NO_2.$$

EXEMPLE 3

Le composé hydroxylé utilisé est un produit d'addition de l'alcool tridécylique sur 6 moles d'oxyde d'éthylène (SURFAROX TD 6, vendu par la Société PROTEX) répondant à la formule :

$$C_{13}H_{27}\left( O-CH_2-CH_2 \right)_6 OH.$$

Dans des conditions opératoires identiques à celles de l'exemple 1, on effectue la nitration de 46,4 g de SURFAROX TD 6 (0,1 mole) ; on récupère 40,7 g de produit, soit un rendement réactionnel de 80 % environ.

Le spectre infra-rouge et la teneur en azote du nitrate obtenu confirment la nature du produit attendu.

$$C_{13}H_{27}\left( O-CH_2-CH_2 \right)_6 O-NO_2.$$

EXEMPLE 4

Le composé hydroxylé utilisé est le produit d'addition d'une coupe d'alcools en $C_{12}-C_4$ sur 4 moles d'oxyde d'éthylène (ALFOL 1214-4 EO vendu par la Société CONDEA), représenté par la formule :

$$\underset{45\ \%}{(C_{12}H_{25}}/\underset{55\ \%}{C_{14}H_{29})}\left( OCH_2-CH_2 \right)_4 OH$$

Dans des conditions opératoires identiques à celles de l'Exemple 1, on effectue la nitration de 37,7 g d'ALFOL 1214-4EO (0,1 mole) ; on récupère 30,4 g de produit, soit un rendement de 72 % environ.

EXEMPLE 5

Le composé hydroxylé utilisé est un produit d'addition d'une coupe d'alcools en $C_{12}$-$C_{14}$ sur 7 moles d'oxyde d'éthylène (ALFOL 1214-7EO vendu par la Société CONDEA) représenté par la formule :

$$(C_{12}H_{25}/C_{14}H_{29})-(O-CH_2-CH_2)_7OH$$

45 % 55 %

La nitration de 50,9 g d'ALFOL 1214-7EO (0,1 mole) fournit 43,2 g de produit, soit un rendement de 78 % environ.

EXEMPLE 6

Le composé hydroxylé utilisé est un produit d'addition d'une coupe d'alcools en $C_{12}$-$C_{14}$ sur 9 moles d'oxyde d'éthylène (ALFOL - 1214 - 9 EO vendu par la Société CONDEA) représenté par la formule :

$$(C_{12}H_{25}/C_{14}H_{29})-(O-CH_2-CH_2)_9OH$$

45 % 55 %

La nitration de 59,7 g d'ALFOL - 1214 -9EO (0,1 mole, fournit 52,6 g de produit, ce qui correspond à un rendement d'environ 82 %.

Les spectres infra-rouge et les teneurs en azote des nitrates obtenus dans les exemples 4, 5 et 6, confirment la nature des produits attendus, à savoir :

$$(C_{12}H_{25}/C_{14}H_{29})-(O-CH_2-CH_2)_4O-NO_2$$ (Exemple 4)

45 % 55 %

$$(C_{12}H_{25}/C_{14}H_{29})-(O-CH_2-CH_2)_7O-NO_2$$ (Exemple 5)

45 % 55 %

$(C_{12}H_{25}/C_{14}H_{29})-(O-CH_2-CH_2)_9 O-NO_2$ (Exemple 6)

45 % 55 %

EXEMPLE 7

.Le composé hydroxylé utilisé est un produit d'addition du nonylphénol sur 2 moles d'oxyde d'éthylène (CEMULSOL NP 2 vendu par la Société SFOS) représenté par la formule :

$C_9H_{19}-\varphi-(O-CH_2-CH_2)_2 OH$

La nitration de 30,9 g de CEMULSOL NP 2 (0,1 mole), fournit 30,7 g de produit, ce qui correspond à un rendement d'environ 87 %.

EXEMPLE 8

Le composé hydroxylé utilisé est un produit d'addition du nonylphénol sur 4 moles d'oxyde d'éthylène (CEMULSOL NP 4 vendu par la Société SFOS) représenté par la formule :

$C_9H_{19}-\varphi-(O-CH_2-CH_2)_4 OH$

A partir de 39,7 g de CEMULSOL NP 4 (0,1 mole), on obtient 33,6 g de produit, soit un rendement de 76 % environ.

EXEMPLE 9

Le composé hydroxylé utilisé est un produit d'addition du nonylphénol sur 9 moles d'oxyde d'éthylène (CEMULSOL NP 9 vendu par la Société SFOS) représenté par la formule :

$C_9H_{19}-\varphi-(O-CH_2-CH_2)_9 OH$

A partir de 61,7 g de CEMULSOL NP 9 (0,1 mole), on obtient 47,7 g de produit, soit un rendement de 72 % environ.

Les spectres infra-rouge et les teneurs en azote des nitrates obtenus dans les Exemples 7, 8 et 9 confirment la nature des produits attendus, à savoir :

$C_9H_{19}-\varphi-(O-CH_2-CH_2)_2-O-NO_2$ (Exemple 7)

$C_9H_{19}-\varphi-(O-CH_2-CH_2)_4-O-NO_2$ (Exemple 8)

$C_9H_{19}-\varphi-(O-CH_2-CH_2)_9-O-NO_2$ (Exemple 9)

EXEMPLE 10

La nitration de CEMULSOL LA 40, décrit dans d'Exemple 1, est de nouveau réalisée en utilisant l'acide sulfurique comme agent de déshydratation le mélange nitrant étant alors composé de 2,25 moles d'acide nitrique et de 1 mole d'acide sulfurique à 98 %.

Le rendement réactionnel observé dans ces conditions est voisin de celui mesuré en présence d'anhydride acétique, et le spectre infra-rouge ainsi que la teneur en azote du produit obtenu confirment la nature du produit attendu.

TESTS SUR LES PRODUITS

On détermine les divers effets sur les gazoles des produits préparés comme décrit dans les Exemples 1 à 10.

TEST A

L'aptitude des nitrates de l'invention à augmenter l'indice de cétane (noté I.C. et déterminé selon la procédure ASTM D-613) des gazoles est mesurée, en comparaison avec des additifs commerciaux, dans un carburant constitué d'un mélange de 80 % de gazole de distillation directe et de 20 % de gazole de craquage catalytique. Les résultats obtenus à différentes concentrations exprimées en ppm poids d'azote d'origine nitrate, sont mentionnés dans le tableau I ci-après.

TABLEAU I

| ADDITIF | ppm d'azote nitrate | I.C. |
|---|---|---|
| Néant | - | 41 |
| Nitrate d'octyle<br>(additif commerciel) | 50<br>100<br>200<br>500 | 40<br>41,5<br>43,3<br>44,3 |
| $C_6H_{13}/C_8H_{17}(OCH_2-CH_2)_2O-NO_2$<br>(produit de l'exemple 1) | 50<br>100<br>200<br>500 | 41<br>42<br>44<br>47 |
| $C_{12}H_{25}(O-CH_2-CH_2)_4O-NO_2$<br>(Produit de l'exemple 2) | 50<br>100<br>200<br>500 | 45,5<br>46,5<br>48<br>54,5 |
| $C_{13}H_{27}(O-CH_2-CH_2)_6O-NO_2$<br>(Produit de l'exemple 3) | 50<br>100<br>200<br>500 | 44,5<br>45,9<br>47,7<br>49,2 |
| $(C_{12}H_{25}/C_{14}H_{29})(O-CH_2-CH_2)_4O-NO_2$<br>45 % 55 %<br>(produit de l'exemple 4) | 50<br>100<br>200<br>500 | 46,5<br>47,8<br>49,8<br>54 |
| $C_9H_{19}-\varphi(O-CH_2-CH_2)_2O-NO_2$<br>(Produit de l'exemple 7) | 50<br>100<br>200<br>500 | 46<br>45,3<br>45<br>47,9 |

Les résultats obtenus montrent qu'à nombre de fonctions nitrates identique, les nitrates des exemples 1 à 4 et 7 ont une aptitude à augmenter l'indice de cétane du gazole sensiblement plus grande que celle d'un nitrate commercial.

TEST B

L'aptitude des nitrates de l'invention à diminuer la tension superficielle du gazole (propriété détergente) est évaluée par mesure de la tension interfaciale entre le gazole additivé et l'eau par la méthode de la goutte pendante.

Cette méthode consiste à photographier le profil méridien d'une goutte de gazole dans l'eau ; l'interprétation mathématique du profil de cette goutte permet, connaissant la différence de densité des liquides, de mesurer la tension interfaciale. Cette méthode a été décrite notamment par S. FORDAM dans Proc. of the Roy. Soc. of London A vol. 194, P 1 (1948) et par E. LEFEBVRE du PREY. dans REVUE INSTITUT FRANCAIS DU PETROLE, XXIV - 6, P 701 (1969). On a utilisé le même mélange de gazoles que dans le Test A.

Les résultats obtenus avec un additif commercial et des additifs de l'invention à différentes concentrations sont mentionnés dans le Tableau II ci-après.

- T A B L E A U II -

| Additif | ppm d'Additif | Tension superficielle mesurée (Newton $10^{-3}$/m) |
|---|---|---|
| Néant | - | 22,2 |
| Nitrate d'isoamyle (additif commercial) | 1 000<br>2 000<br>3 000 | 19,9<br>21<br>21 |
| $C_{12}H_{25}(O-CH_2-CH_2)_4O-NO_2$ (produit de l'exemple 2) | 1 000<br>2 000<br>3 000 | 15,2<br>13,3<br>10,9 |
| $C_{13}H_{27}(O-CH_2-CH_2)_6O-NO_2$ (produit de l'exemple 3) | 1 000<br>2 000<br>3 000 | 11,3<br>9<br>6,5 |
| $(C_{12}H_{25}/C_{14}H_{29})(O-CH_2-CH_2)_4O-NO_2$<br>45 % 55 %<br>(produit de l'exemple 4) | 1 000<br>2 000<br>3 000 | 11,8<br>10<br>8,2 |
| $C_9H_{19}-\varphi(O-CH_2-CH_2)_2O-NO_2$ (produits de l'exemple 7) | 1 000<br>2 000<br>3 000 | 20,1<br>19<br>18,3 |

Les résultats obtenus montrent que les propriétés tensio-actives des nitrates de l'invention, notamment les nitrates d'alcools en $C_{12}$, $C_{13}$ et $C_{12}C_{14}$ éthoxylés, sont très nettement supérieures à celles d'un additif "pro-cétane" commercial .

TEST C

On détermine l'aptitude des produits de l'invention à supprimer les inconvénients liés à l'encrassement des systèmes d'injection.

On alimente un véhicule du type voiture particulière avec un gazole renfermant 78 % d'une coupe pétrolière de distillation directe et 22 % de L.C.O. Le véhicule est disposé sur un chassis dynamométrique et effectue un parcours d'environ 2500 km selon un cycle comportant des phases à vitesse stabilisée à 50 km/h. Avant et après ce parcours, on effectue des cycles urbains du type ECE (Le cycle ECE est utilisé en Europe pour mesurer les émissions de polluants et la consommation de carburant des véhicules).

Les essais ont été réalisés avec le gazole seul et avec le même gazole contenant 2000 ppm de l'additif de l'Exemple 4.

Les résultats relatifs aux émissions de polluants sont présentés dans le Tableau III ci-après.

- T A B L E A U III -

| Gazole | Gazole de Réf. (sans additif) | | Gazole + 2000 ppm du nitrate de l'Exemple 4 | |
|---|---|---|---|---|
| Kilomètrage réalisé avec un lot donné d'injecteurs | 0 | 2500 km | 0 | 2500 km |
| Emissions selon le cycle ECE : | | | | |
| CO (g/essai) | 6,4 | 10,8 | 6,6 | 6,8 |
| Hydrocarbures " imbrûlés | 1,6 | 6,9 | 1,4 | 1,3 |
| Particules " | 0,4 | 1,5 | 0,5 | 0,6 |

Ces résultats montrent, pour le gazole non additivé, un accroissement très net des taux de polluants entre le début de l'essai et après 2500 km. On observe corrélativement une détérioration très importante du fonctionnement au ralenti et à faible charge (bruit, régime instable).

Avec le gazole additivé, l'évolution des taux de polluants entre le début de l'essai et après 2500 km est très faible. En outre le moteur fonctionne régulièrement et sans bruit anormal.

REVENDICATIONS

1. Composé organique à fonction nitrate, caractérisé en ce qu'il répond à la formule générale :

$$R \leftarrow O-CH_2-CHX \rightarrow_n O-NO_2 \quad (I)$$

dans laquelle R représente un radical alkyle, linéaire ou ramifié, de 6 à 20 atomes de carbone ou un radical aryle, substitué par une ou plusieurs chaînes alkyles de 4 à 18 atomes de carbone ; X est un atome d'hydrogène ou un groupe méthyle et n est un nombre entier de 1 à 15.

2. Composé organique selon la revendication 1, caractérisé en ce que, dans le formule (I), n est un nombre entier de 2 à 10.

3. Composé organique selon la revendication 1 ou 2, caractérisé en ce qu'il comprend, dans la même molécule, des groupements $-O-CH_2-CHX-$ dans lesquels X est un atome d'hydrogène, et des groupements $-O-CH_2-CHX-$ dans lesquels X est un radical méthyle.

4. Composition caractérisée en ce qu'elle consiste en un mélange de composés selon l'une des revendications 1 à 3, dans lesquels les radicaux alkyles R différent par leur nombre d'atomes de carbone.

5. Procédé de préparation d'un composé organique selon l'une des revendications 1 à 3 ou d'une composition selon la revendication 4, caractérisé en ce que l'on réalise la nitration d'au moins un composé hydroxylé répondant à la formule générale :

$$R \leftarrow O-CH_2-CHX \rightarrow_n OH$$

au moyen d'acide nitrique à une température de - 10°C à + 30°C, et en présence d'un agent déshydratant choisi parmi l'anhydride acétique et l'acide sulfurique, l'excès d'acidité des produits obtenus étant neutralisé au moyen d'une base minérale.

6. Composition de carburant pour moteurs Diesel, caractérisée en ce

qu'elle contient une proportion majeure d'au moins un gazole et une proportion mineure, suffisante pour en élever l'indice de cétane, d'au moins un composé selon l'une des revendications 1 à 3.

7. Composition de carburant pour moteurs Diesel selon la revendication 6, caractérisée en ce que la proportion dudit (desdits) composé(s) est de 0,01 à 3 % en poids par rapport au(x) gazole(s).

8. Composition de carburant selon l'une des revendications 6 et 7, caractérisée en ce que ledit gazole comprend au moins une coupe moyenne provenant de la conversion de fractions lourdes de pétrole.

9. Composition de carburant selon l'une des revendications 6 à 8, caractérisée en ce que ledit gazole comprend au moins une coupe de distillation directe de pétrole brut à caractère aromatique.

10. Composition de carburant selon l'une des revendications 6 à 9, caractérisée en ce que ledit gazole contient en outre une proportion mineure d'au moins un composé oxygéné choisi parmi les alcools, les cétones et les éthers.

0157684

Office européen des brevets

# RAPPORT DE RECHERCHE EUROPEENNE

Numéro de la demande

EP 85 40 0499

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-2 742 492 (C.C. HARVEN)<br>* Colonne 1, lignes 15-27; colonne 2, lignes 32-36,48-53 et 57-61 *<br>--- | 1,5,6 | C 07 C 77/02<br>C 07 C 76/04<br>C 10 L 1/22 |
| Y | DE-A-2 039 609 (COUNSIL OF SCIENTIFIC AND INDUSTRIAL RESEARCH)<br>* Page 1; page 2, alinéa 2 *<br>----- | 1,5,6 | |
| | | | DOMAINES TECHNIQUES RECHERCHES (Int. Cl.4) |
| | | | C 07 C 77/00<br>C 07 C 76/00 |

Le présent rapport de recherche a été établi pour toutes les revendications

| Lieu de la recherche | Date d'achèvement de la recherche | Examinateur |
|---|---|---|
| LA HAYE | 21-05-1985 | KINZINGER J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82